# EUROPÄISCHE PATENTSCHRIFT

(11) **EP 1 268 028 B1**
(45) Veröffentlichungstag und Bekanntmachung des Hinweises auf die Patenterteilung: **13.10.2004**
(21) Anmeldenummer: 01923601.7
(22) Anmeldetag: 22.02.2001
(51) Int. Cl.: B01D 24/46, A61L 2/18, C01B 11/14

(54) **VERFAHREN ZUM ENTKEIMENDEN UND REINIGENDEN SPÜLEN PARTIKELHALTIGER FILTERBETTEN**
METHOD FOR FLUSHING PARTICLE-BEARING FILTER BEDS, TO STERILISE AND DECONTAMINATE THE SAME
PROCEDE DE LAVAGE NETTOYANT ET DESINFECTANT DE LITS FILTRANTS A PARTICULES

(30) Priorität: 02.03.2000 DE 10010255
(43) Veröffentlichungstag der Anmeldung: 02.01.2003
(73) Patentinhaber: P & W Invest Vermögensverwaltungsgesellschaft MbH, 5020 Salzburg (AT)
(72) Erfinder: POLAK, Walter, A-5020 Salzburg (AT)
(74) Vertreter: Hagemann, Heinrich, Dr.rer.nat., Dipl.-Chem.
(86) Internationale Anmeldenummer: PCT/EP2001/002030
(87) Internationale Veröffentlichungsnummer: WO 2001/064310

(56) Entgegenhaltungen:
- EP-A- 0 104 341
- BE-A- 853 566
- DE-A- 3 403 631

## Beschreibung

Die Erfindung betrifft ein Verfahren zum entkeimenden und reinigenden Spülen partikelhaltiger Filterbetten von cyclischen oder linearen Wasseraufbereitungsanlagen.

Bei der Aufbereitung von Wasser in cyclischen oder linearen Aufbereitungsanlagen stellt das Filtern des Wassers einen bedeutenden und grundlegenden Verfahrensschritt dar. Dabei sollen in erster Linie feste Stoffe, die sich im unfiltrierten Wasser befinden, vom Filterbett zurückgehalten werden. Das Filterbett besteht in der Regel aus Materialien, wie Kieskörnern, Sand, Schamott, Zeolithen oder kohlehaltigen Materialien, an oder zwischen denen sich feste Stoffe oder geflockte Substanzen absetzen. Diese Ablagerung macht es erforderlich, das Filterbett in regelmäßigen Zeitabständen zu reinigen. In der Regel, aber nicht bei allen Systemen, wird dabei Wasser verwendet, das in im Vergleich zur Strömungsrichtung des zu filtrierenden Wassers beim Filtrationsvorgang entgegengesetzter Richtung durch den Filter gepumpt wird. Man bezeichnet diesen Vorgang als Spülen bzw. Filterspülen. Zum Spülen können auch Luft und Wasser-Luft-Wasser-Gemische jeweils einzeln oder in Kombination miteinander eingesetzt werden.

Neben der Bildung von Ablagerungen organischer oder anorganischer Herkunft kommt es mit zunehmender Betriebszeit des Filterbettes häufig auch zu einer Kontamination der Filtermaterialien mit Mikroorganismen, wobei auch pathogene Keime anzutreffen sind. Untersuchungen haben gezeigt, daß etwa 80 bis 90% der Filtermaterialien, insbesondere im unteren Bereich der Filtermaterialschicht, in hohem Maße durch Keime und biogene Ablagerungen (Biofilme) belastet sind. Neben dem Filtermaterial sind davon auch die Filtratkammerflächen sowie die Filterdüsen und auch die Wandungen des Spülwasserbeckens betroffen. Daraus können sich erhebliche Probleme hinsichtlich der Hygiene z.B. des Schwimm- und Badebeckenwassers ergeben - d.h. obwohl in diesem Fall der Badegast nur mit dem Beckenwasser unmittelbar in Berührung kommt, stellt eine Kontamination des Filters mit Mikroorganismen ein erhebliches Risiko dar, da z.B. bei plötzlich absinkender keimtötender Kapazität des Badewassers in kurzer Zeit große Mengen von Keimen aus dem befallenen Filter ins Badewasser gelangen können.

Die DE-A-32 33 857 beschreibt ein Verfahren und eine Einrichtung zum Reinigen von Filterelementen, wobei ein Gemisch aus Wasser und einem vorgespannten Gas verwendet wird. Gegebenenfalls wird der Gasanteil des Gemisches mit einem Netzmittel angereichert, um die Oberflächenspannung des Wasser herabzusetzen, so daß ein homogeneres Spülmittelgemisch erhalten wird.

Die DE-32 29 219. beschreibt die Verwendung einer grenzflächenaktiven Substanz als Rückspülmittel für partikelhaltige rückspülbare Filterbetten zur Wasseraufbereitung bei Schwimmbad-, Trink- und Abwasseraufbereitungsanlagen. Bei diesem Verfahren ist die Reinigungswirkung gegenüber festen Ablagerungen jedoch nur mäßig. Insbesondere wird nur eine geringe Wirkung gegenüber Kontaminationen des Filters mit Mikroorganismen und biogenen Ablagerungen erzielt. Darüber hinaus muß die grenzflächenaktive Substanz in hohen Konzentrationen eingesetzt werden, was zu hohen Kosten und zu einer hohen Belastung des Abwassers führt.

Die BE-A-853 566 beschäftigt sich mit einer Vorrichtung und einem Verfahren zum Desinfizieren von Filterbetten, insbesondere bei Ionenaustauschern, die bei der Wasseraufbereitung eingesetzt werden. Hierbei ist ein hoher apparativer Aufwand für den Aufbau eines zusätzlichen Kreislaufs erforderlich, wobei sehr hohe Wassermengen zum Einsatz kommen. Die beschriebenen Desinfektionsmittel in Form von Halogenen, wie Brom, Chlor und Iod, sowie Verbindungen des Chlors, Nitraten und quaternären Ammoniumverbindungen scheinen keine vollständige Entkeimung bzw. Reinigung zu ermöglichen. Die DE-34 03 631 C2 bezieht sich auf ein Verfahren zur Herstellung einer wässrigen Chloritlösung, die zur Wasseraufbereitung herangezogen werden kann. Hierzu wird die Chloritlösung mittels einer Feindosierpumpe unmittelbar in den Beckenwasserkreislauf dosiert, d.h. direkt dem Schwimmbadwasser zugesetzt. Da im Hinblick auf die im Schwimmbadwasser Badenden nur sehr geringe Konzentrationen eingesetzt werden können, ist eine Entkeimungs- bzw. Reinigungswirkung des Filters nicht sichergestellt. Auch erfordert die Reinigung des Filters in diesem dynamischen System einen hohen Wasserdurchsatz, wodurch eine Verschleppung von aus dem Filter herausgeschwemmten Mikroorganismen in den Schwimmbadkreislauf und damit Kontaminierung des Schwimmbadwassers möglich ist.

Es war daher Aufgabe der vorliegenden Erfindung, ein Verfahren bereitzustellen, das die vorstehend genannten Nachteile vermeidet. Insbesondere sollte das erfindungsgemäße Verfahren neben einer hohen Reinigungswirkung gegenüber organischen und anorganischen Festablagerungen im Filterbett auch eine wirksame Dekontamination des gesamten Filters bezüglich Mikroorganismen und biogenen Ablagerungen erlauben und gleichzeitig kostengünstig sein sowie zu nur geringen Belastungen des Abwassers führen.

Erfindungsgemäß wird diese Aufgabe durch ein Verfahren zum entkeimenden und reinigenden Spülen partikelhaltiger Filterbetten von cyclischen oder linearen Wasseraufbereitungsanlagen gelöst, welches dadurch gekennzeichnet ist, dass der Filter mit einer chloroxid- und/oder halogenhaltigen und/oder peroxidhaltigen wässrigen Lösung beschickt wird, die man auf die Ablagerung des partikelförmigen Fitterbettes einwirken lässt und danach zum Entfernen der prozessbedingten Reaktionsprodukte sowie verbliebenen Chloroxids, Halogens und/oder Peroxids mit Wasser oder einem wässrigen Medium spült, wobei es sich bei dem partikelhaltigen Filterbett um einen Ein-/Mehrschichtfilter handelt und die Filtermaterialien Quarz, Kies, Sand, poröse Filtermaterialien und/oder kohlehaltige Materialien beinhalten.

Wesentliches Merkmal des erfindungsgemäßen Verfahrens ist demzufolge eine zweistufige Vorgehensweise. Im ersten Verfahrensschritt wird der Filter mit der chloroxid- und/oder halogenhaltigen und/oder peroxidhaltigen wäßrigen Lösung beschickt, die man anschließend auf das Filtermaterial einwirken läßt. Bei dem zweiten Verfahrensschritt erfolgt dann das Spülen des Filters mit Wasser bzw. einem wäßrigen Medium.

Das erfindungsgemäße Verfahren eignet sich besonders zur Anwendung in cyclischen Wasseraufbereitungsanlagen, wie Schwimmbadkreisläufen, Kühlwasserkreisläufen und Brauchwasserkreisläufen, und linearen Wasseraufbereitungsantagen, wie Trinkwasserund Abwasseraufbereitungsanlagen.

Als besonders vorteilhaft hat es sich erwiesen, daß man die chloroxid- und/oder halogenhaltige und/oder peroxidhaltige wäßrige Lösung für etwa 1 bis 3 Stunden auf die Ablagerungen des partikelförmigen Filterbettes einwirken läßt. Unter bestimmten Bedingungen sind kürzere Intervalle ausreichend bzw. längere notwendig.

Unter den Chloroxiden ist sowohl aus Gründen der Wirksamkeit als auch aus Gründen der Zugänglichkeit das Chlordioxid besonders bevorzugt, so daß es sich bei der im erfindungsgemäßen Verfahren eingesetzten chloroxidhaltigen Lösung vorzugsweise um eine chlordioxidhaltige wäßrige Lösung handelt. Es hat sich gezeigt, daß die oxidativen Eigenschaften des Chlordioxids zu einer hohen Wirksamkeit des erfindungsgemäßen Verfahrens führen. Da es sich bei Chlordioxid um eine chemisch instabile und schwer handhabbare Substanz handelt, ist es vorteilhaft, daß ein großer Teil des Chlordioxids in der Lösung nicht in freier Form vorliegt, sondern chemisch gebunden in Form einer chemischen Spezies, aus der Chlordioxid stetig nachgebildet wird. Eine solche Substanz ist das Tetrachlordecaoxid-Komplex-Dianion [Cl₄O₁₀]²⁻, das unter der ELINCS-Nr. 420-970-2 dokumentiert ist. Das Tetrachlordecaoxid-Komplex-Dianion steht mit dem Chlordioxid in einem Gleichgewicht, so daß eine Lösung des Tetrachlordecaoxid-Komplex-Dianions stets eine gewisse Menge an Chlordioxid enthält, das bei dessen Verbrauch nachgebildet wird.

In einer bevorzugten Ausführungsform liegt daher in der chlordioxidhaltigen wäßrigen Lösung das Tetrachlordecaoxid-Komplex-Dianion [Cl₄O₁₀]²⁻ vor.

Die Tetrachlordecaoxid-Komplex-Dianionen und Chlordioxid enthaltende Lösung wird vorzugsweise erhalten, indem eine einen pH-Wert ≤ 3 aufweisende, sulfationenhaltige wäßrige Lösung mit einer darin stabilen Peroxyverbindung versetzt wird und diese Lösung anschließend mit der wäßrigen. alkalischen Lösung eines Chlorits vermischt wird. Die sulfationenhaltige wäßrige Lösung wird vorzugsweise mit einer Menge einer darin stabilen Peroxyverbindung versetzt, die eine Konzentration an Peroxyverbindungen im Endprodukt von etwa 0,001 bis 0,01 molar ergibt. Als besonders vorteilhaft hat es sich erwiesen, die sulfationenhaltige wäßrige Lösung mit einer darin stabilen Peroxyverbindung zu versetzen und diese Lösung anschließend mit der wäßrigen, alkalischen Lösung eines Chlorits in einer Menge zu vermischen, daß ein pH-Wert von über 7,0, insbesondere zwischen 7,5 und 8,0 eingestellt wird.

Je nach Grad der Verschmutzung und/oder der bakteriellen Kontamination des Filters kann das erfindungsgemäße Verfahren mit unterschiedlichen Konzentrationen der beschriebenen Tetrachlordecaoxid-Komplex-Dianionen und Chlordioxid enthaltenden Lösungen angewendet werden. In einer bevorzugten Ausführungsform läßt man die Lösung in einer Konzentration auf die Ablagerungen des partikelförmigen Filterbettes einwirken, die einer Anfangskonzentration an Chlorit von etwa 5 bis 20 mmol/l entspricht.

Als besonders vorteilhaft hat es sich erwiesen, als Peroxyverbindung eine anorganische Peroxyverbindung in Form von Wasserstoffperoxid, eines Persulfats, Percarbonats. Perborats oder eines Peroxids eines Alkali- oder Erdalkalimetalls zu verwenden. Als Chlorit wird vorzugsweise ein Alkali- und/oder Erdalkalichlorit eingesetzt. Besonders vorteilhafte Ergebnisse stellen sich dann ein, wenn die sulfationenhaltige wäßrige Lösung einen pH-Wert ≤ 1 aufweist.

Wenn die eingesetzten wäßrigen Ausgangslösungen lediglich eine geringe Carbonathärte zeigen bzw. mit demineralisiertem Wasser hergestellt werden, empfiehlt es sich, unter einer Inertgasatmosphäre zu arbeiten, da es in Abwesenheit einer Schutzgasschicht zwischen dem geringfügig ausgasenden Chlordioxid und Luft zur Bildung von explosiven Luft/Chlordioxidgemischen kommen kann. Luft/Chlordioxidgemische neigen bei einem Verhältnis der Bestandteile Luft zu Chlordioxid von etwa 10:1 zu einem explosionsartigen Zerfall des Chlordioxids in Chlor und Sauerstoff Bei wäßrigen Ausgangslösungen mittlerer bzw. hoher Carbonathärte ( > etwa 7 Grad bzw. > etwa 1,3 mmol/l Carbonathärte) ist der Einsatz eines Inertgases im allgemeinen nicht nötig, da sich eine Art Schutzgasschicht aus Kohlendioxid bildet. Vorzugsweise wird daher zur Herstellung der sulfationenhaltigen wäßrigen Lösung, die zur Bildung der Tetrachlordecaoxid-Komplex-Dianionen und Chlordioxid enthaltenden Lösung eingesetzt werden soll, mineralisiertes Wasser verwendet.

Vorzugsweise enthält die Tetrachlordecaoxid-Komplex-Dianion und Chlordioxid enthaltende Lösung ein wasserlösliches Phosphat, da die Menge an Peroxyverbindungen herabgesetzt werden kann, wenn der Fertiglösung ein wasserlösliches Phosphat, so z.B. Natriummetapolyphosphat, in geringer Menge, einverleibt wird.

Das erfindungsgemäße Verfahren eignet sich zum entkeimenden und reinigenden Spülen partikelhaltiger Filterbetten verschiedener Typen in cyclischen und linearen Wasseraufbereitungsanlagen. Es bestehen keine prinzipiellen Einschränkungen hinsichtlich der Art, Größe und des Aufbaus der Filterbetten. Bei den partikelhaltigen Filterbetten handelt es sich um Ein-/Mehrschichtfilter, die Filtermaterialien aus Quarz, Kies und/oder Sand und/oder porösen Filtermaterialien, wie Schamotten. Zeolithen, und/oder kohlehaltigen Materialien, wie Aktivkohle, Braunkohle und Antrazithkohle , beinhalten. Sollte eine besonders gründliche Entfernung von Chlor und/oder Chlordioxid im zweiten Verfahrensschritt angestrebt werden, so kann zum Spülen an Stelle von Wasser eine wäßrige Lösung eines Thiosulfats (beispielsweise Natriumthiosulfat) verwendet werden. Dabei werden vorteilhafterweise pro Gramm Chlor etwa 5,3 g Thiosulfat und pro Gramm Chlordioxid etwa 3,0 g Thiosulfat eingesetzt.

Zusammenfassend läßt sich feststellen, daß das erfindungsgemäße Verfahren ein entkeimendes und reinigendes Spülen partikelhaltiger Filterbetten von cyclischen und linearen Wasseraufbereitungsanlagen ermöglicht, das im Vergleich zum Stand der Technik zu stark verbesserten Ergebnissen führt. So ist die Reinigungswirkung gegenüber festen organischen und anorganischen Ablagerungen deutlich verbessert. Besonders ausgeprägt sind die Vorteile hinsichtlich der Wirkung gegenüber Kontaminationen durch Mikroorganismen und biogenen Ablagerungen (Biofilme). Die im Stand der Technik beschriebenen Reinigungsverfahren zeigen hier nur eine schwache Wirkung. Überraschend hat sich gezeigt, daß das erfindungsgemäße Verfahren nicht nur zu einer weitgehenden Dekontamination der behandelten Filterbetten bzgl. eines Befalls mit Mikroorganismen und biogenen Ablagerungen führt, sondern, insbesondere bei porösen Filtermaterialien, auch eine starke vorbeugende Wirkung erzielt. Dies ist möglicherweise darauf zurückzuführen, daß durch das Abtöten sämtlicher Keime und die gleichzeitige Beseitigung aller Verunreinigungen, die das Wachstum der Mikroorganismen fördern könnten, indem sie z.B. als Nahrungsquelle dienen, ein Ausbreiten von Mikroorganismen auch für einen längeren Zeitraum nach der Behandlung verhindert wird. Der Einsatz des erfindungsgemäßen Verfahrens kann daher als vorbeugende Maßnahme gesehen und durchgeführt werden. Es ist aber auch im Sinne einer "Sofortmaßnahme" für die "Sanierung" von Filtermaterial geeignet. Darüber hinaus ist das erfindungsgemäße Verfahren nur mit geringen Kosten verbunden und führt nicht zu einer nennenswerten Belastung des Abwassers.

Nachfolgend soll die Erfindung anhand von Beispielen noch näher erläutert werden:

### Beispiel 1: Herstellung einer Tetrachlordecaoxid-Komplex-Dianionen und Chlordioxid enthaltenden Lösung

In 1 1 sulfathaltiges Wasser (Carbonathärte: 18 Grad bzw. 3,2 mmol/l) eines pH-Wertes von 0,5 werden 0,5 g einer 30 gew.-%igen Wasserstoffperoxidlösung gegeben. Zu dieser Lösung werden unter gutem Rühren 0,9 l einer handelsüblichen Natriumchloritlösung (etwa 300 g Natriumchlorit/l) hinzugefügt. Dabei durchläuft die Lösung eine braune Färbung, die beim Überschreiten des pH-Wertes von 7 nach Ablauf der Stabilisierungsreaktion in eine helle lindgrüne Farbe umschlägt. Hierbei stellt sich in der Lösung ein pH-Wert von 7,5 ein.

### Beispiel 2: Behandlung eines Mehrschichtfilters mit Kohlematerialauflage zur Reinigung von Schwimm- und Badebeckenwasser mit dem erfindungsgemäßen Verfahren

Bei dem für dieses Beispiel herangezogenen Filter handelt es sich um einen zur Reinigung von Badewasser im Bereich von Frei- und Hallenbädern typischen Filter. Er ist durch die folgenden Kenngrößen charakterisiert:
- Höhe:: 2,5 m
- Durchmesser:: 2,1 m
- Volumen des Filterbetts:: 5,2 m³
- Material der Filterhülle:: Glasfaserverstärkter Kunststoff
- Material der Filterbeschickung:: 4 Quarzkiesschichten mit nach oben sinkender Korngröße sowie eine oberste Filterschicht aus Kornkohlematerial

Das erfindungsgemäße Spülverfahren wurde wie folgt auf den beschriebenen Filter angewendet:

Zunächst wird der Filter mit Wasser normal gespült und anschließend wird der Filterbehälter bzgl. des Wasser entleert. Anschließend werden 6,7 l der in Beispiel 1 beschriebenen Tetrachlordecaoxid-Komplex-Dianion und Chlordioxid enthaltenden Lösung mittels einer Dosierpumpe von unten beginnend in den Filter eingebracht. Daraufhin wird der Filter von unten vollständig mit Wasser befüllt. Nach vollständiger Beschickung des Filters läßt man etwa 1,5 h einwirken. Nach dieser Einwirkzeit erfolgt ein Spülen des Filters mit Wasser bei einer Spülgeschwindigkeit von etwa 40 m/h für etwa 10 min.

## Patentansprüche

1. Verfahren zum entkeimenden und reinigenden Spülen partikelhaltiger Filterbetten von cyclischen oder linearen Wasseraufbereitungsanlagen, **dadurch gekennzeichnet, dass** der Filter mit einer chloroxid- und/oder halogenhaltigen und/oder peroxidhaltigen wässrigen Lösung diskontinuierlich beschickt wird, die man auf die Ablagerungen des partikelförmigen Filterbettes einwirken lässt und danach zum Entfernen der prozessbedingten Reaktionsprodukte sowie verbliebenen Chloroxids, Halogens und/oder Peroxids mit Wasser oder einem wässrigen Medium spült, wobei es sich bei dem partikelhaltigen Filterbett um einen Ein-/Mehrschichtfilter handelt und die Filtermaterialien Quarz, Kies, Sand, poröse Filtermaterialien und/oder kohlehaltige Materialien beinhalten.

2. Verfahren nach Anspruch 1, **dadurch gekennzeichnet, daß** man die chloroxidund/oder halogenhaltige und/oder peroxidhaltige wäßrige Lösung für etwa 1 bis 3 Stunden auf die Ablagerungen des partikelförmigen Filterbettes einwirken läßt.

3. Verfahren nach Anspruch 1 oder 2, **dadurch gekennzeichnet, daß** es sich bei der chloroxidhaltigen wäßrigen Lösung um eine chlordioxidhaltige wäßrige Lösung handelt.

4. Verfahren nach Anspruch 3, **dadurch gekennzeichnet, daß** in der chlordioxidhaltigen wäßrigen Lösung das Tetrachlordecaoxid-Komplex-Dianion [Cl₄O₁₀]²⁻ vorliegt.

5. Verfahren nach Anspruch 3 oder 4, **dadurch gekennzeichnet, daß** die chlordioxidhaltige wäßrige Lösung erhalten wird, indem eine einen pH-Wert ≤ 3 aufweisende, sulfationenhaltige wäßrige Lösung mit einer darin stabilen Peroxyverbindung versetzt und diese Lösung anschließend mit der wäßrigen, alkalischen Lösung eines Chlorits vermischt wird.

6. Verfahren nach Anspruch 5, **dadurch gekennzeichnet, daß** die sulfationenhaltige wäßrige Lösung mit einer solchen Menge einer darin stabilen Peroxyverbindung versetzt wird, daß sich eine Konzentration an Peroxyverbindung im Endprodukt von etwa 0,001 bis 0,01 molar ergibt.

7. Verfahren nach mindestens einem der Ansprüche 5 oder 6, **dadurch gekennzeichnet, daß** die sulfationenhaltige wäßrige Lösung mit einer darin stabilen Peroxyverbindung versetzt und diese Lösung anschließend mit der wäßrigen, alkalischen Lösung eines Chlorits in einer Menge vermischt wird, daß ein pH-Wert von über 7,0, insbesondere zwischen 7,5 und 8,0, eingestellt wird.

8. Verfahren nach mindestens einem der Ansprüche 5 bis 7, **dadurch gekennzeichnet, daß** man die chlordioxidhaltige wäßrige Lösung in einer Konzentration auf die Ablagerungen des partikelförmigen Filterbettes einwirken läßt, die einer Anfangskonzentration an Chlorit von etwa 5 bis 20 mmol/l entspricht.

9. Verfahren nach mindestens einem der Ansprüche 5 bis 8, **dadurch gekennzeichnet, daß** als Peroxyverbindung eine anorganische Peroxyverbindung in Form von Wasserstoffperoxid, eines Persulfats, Percarbonats, Perborats oder eines Peroxids eines Alkali- oder Erdalkalimetalls verwendet wird.

10. Verfahren nach mindestens einem der Ansprüche 5 bis 9, **dadurch gekennzeichnet, daß** als Chlorit ein Alkali- und/oder Erdalkalichlorit eingesetzt wird.

11. Verfahren nach mindestens einem der Ansprüche 5 bis 10, **dadurch gekennzeichnet, daß** die sulfationenhaltige wäßrige Lösung einen pH-Wert ≤ 1 aufweist.

12. Verfahren nach mindestens einem der Ansprüche 5 bis 11, **dadurch gekennzeichnet, daß** zur Herstellung der sulfationenhaltigen wäßrigen Lösung mineralisiertes Wasser verwendet wird.

13. Verfahren nach mindestens einem der Ansprüche 5 bis 12, **dadurch gekennzeichnet, daß** die chlordioxidhaltige wäßrige Lösung ein wasserlösliches Phosphat enthält.

## Claims

1. Process for the disinfecting and cleaning washing of particulate filter beds of cyclic or linear water treatment plants, **characterized in that** the filter is charged batchwise with a chlorine-oxide- and/or halogen- and/or peroxide-containing aqueous solution which is allowed to act on the sediments of the particulate filter bed and thereafter, to remove the reaction products of the process and also residual chlorine oxide, halogen and/or peroxide, the filter is washed with water or an aqueous medium, the particulate filter bed being a single-layer/multilayer filter and the filter materials comprising quartz, gravel, sand, porous filter materials and/or carbonaceous materials.

2. Process according to Claim 1, **characterized in that** the chlorine-oxide- and/or halogen-containing and/or peroxide-containing aqueous solution is allowed to act on the sediments of the particulate filter bed for about 1 to 3 hours.

3. Process according to Claim 1 or 2, **characterized in that** the chlorine-oxide-containing aqueous solution is a chlorine-dioxide-containing aqueous solution.

4. Process according to Claim 3, **characterized in that** the tetrachlorodecaoxide complex dianion [Cl₄O₁₀]²⁻ is present in the chlorine-dioxide-containing aqueous solution.

5. Process according to Claim 3 or 4, **characterized in that** the chlorine-dioxide-containing aqueous solution is obtained by admixing a sulphate-ion-containing aqueous solution having a pH ≤ 3 with a peroxy compound stable therein and then mixing this solution with the aqueous alkaline solution of a chlorite.

6. Process according to Claim 5, **characterized in that** the sulphate-ion-containing aqueous solution is admixed with an amount of a peroxy compound stable therein such that a concentration of peroxy compound in the end product of about 0.001 to 0.01 molar results.

7. Process according to at least one of Claims 5 and 6, **characterized in that** the sulphate-ion-containing aqueous solution is admixed with a peroxy compound stable therein and this solution is then mixed with the aqueous alkaline solution of a chlorite in an amount such that a pH greater than 7.0, preferably between 7.5 and 8.0, is set.

8. Process according to at least one of Claims 5 to 7, **characterized in that** the chlorine-dioxide-containing aqueous solution is allowed to act on the sediments of the particulate filter bed at a concentration which is equivalent to an initial chlorite concentration of about 5 to 20 mmol/l.

9. Process according to at least one of Claims 5 to 8, **characterized in that** the peroxy compound is an inorganic peroxy compound in the form of hydrogen peroxide, a persulphate, percarbonate, perborate, or a peroxide of an alkali metal or alkaline earth metal.

10. Process according to at least one of Claims 5 to 9, **characterized in that** the chlorite used is an alkali metal chlorite and/or alkaline earth metal chlorite.

11. Process according to at least one of Claims 5 to 10, **characterized in that** the sulphate-ion-containing aqueous solution has a pH ≤ 1.

12. Process according to at least one of Claims 5 to 11, **characterized in that** mineralized water is used to prepare the sulphate-ion-containing aqueous solution.

13. Process according to at least one of Claims 5 to 12, **characterized in that** the chlorine-dioxide-containing aqueous solution comprises a water-soluble phosphate.

## Revendications

1. Procédé de nettoyage et de stérilisation par lavage de lits filtrants à particules dans des installations cycliques ou en ligne d'épuration d'eau, **caractérisé en ce qu'**on fait passer en discontinu sur le filtre une solution aqueuse contenant de l'oxyde de chlore et/ou un halogène et/ou un peroxyde, que l'on fait agir sur les dépôts retenus par le lit filtrant à particules, après quoi on lave avec de l'eau ou avec un milieu aqueux pour éliminer les produits de réaction résultant du processus ainsi que l'oxyde de chlore, l'halogène et/ou le peroxyde restants, le lit filtrant à particules étant un filtre à une seule couche ou à plusieurs couches et les matières filtrantes consistant en quartz, gravier, sable, matières filtrantes poreuses et/ou matières contenant du charbon.

2. Procédé suivant la revendication 1, **caractérisé en ce qu'**on fait agir la solution aqueuse contenant de l'oxyde de chlore et/ou un halogène et/ou un peroxyde pendant environ 1 à 3 heures sur les dépôts du lit filtrant à particules.

3. Procédé suivant la revendication 1 ou 2, **caractérisé en ce que** la solution aqueuse contenant de l'oxyde de chlore est une solution aqueuse contenant du dioxyde de chlore.

4. Procédé suivant la revendication 3, **caractérisé en ce que** le dianion complexe tétrachlorodécaoxyde [Cl₄O₁₀]²⁻ est présent dans la solution aqueuse contenant du dioxyde de chlore.

5. Procédé suivant la revendication 3 ou 4, **caractérisé en ce que** la solution aqueuse contenant du dioxyde de chlore est obtenue par addition à une solution aqueuse contenant des ions sulfate, présentant un pH de valeur inférieure ou égale à 3, d'un composé peroxy qui y est stable et on mélange ensuite la solution obtenue avec la solution alcaline aqueuse d'un chlorite.

6. Procédé suivant la revendication 5, **caractérisé en ce que** la solution aqueuse contenant des ions sulfate est additionnée d'un composé peroxy qui y est stable en une quantité choisie de manière à obtenir une concentration molaire en composé peroxy d'environ 0,001 à 0,01 dans le produit final.

7. Procédé suivant au moins l'une des revendications 5 ou 6, **caractérisé en ce que** la solution aqueuse contenant des ions sulfate est additionnée d'un composé peroxy qui y est stable et cette solution est ensuite mélangée avec la solution alcaline aqueuse d'un chlorite en une quantité choisie de manière ajustée à un pH de valeur supérieure à 7,0, compris en particulier entre 7,5 et 8,0.

8. Procédé suivant au moins l'une des revendications 5 à 7, **caractérisé en ce qu'**on fait agir la solution aqueuse contenant du dioxyde de chlore sur les dépôts du lit filtrant à particules à une concentration qui correspond à la concentration initiale en chlorite d'environ 5 à 20 mmol/l.

9. Procédé suivant au moins l'une des revendications 5 à 8, **caractérisé en ce qu'**on utilise comme composé peroxy un composé peroxy inorganique sous forme de peroxyde d'hydrogène, d'un persulfate, d'un percarbonate, d'un perborate ou d'un peroxyde, d'un métal alcalin ou d'un métal alcalino-terreux.

10. Procédé suivant au moins l'une des revendications 5 à 9, **caractérisé en ce qu'**on utilise comme chlorite un chlorite de métal alcalin et/ou un chlorite de métal alcalino-terreux.

11. Procédé suivant au moins l'une des revendications 5 à 10, **caractérisé en ce que** la solution aqueuse contenant des ions sulfate présente une valeur de pH inférieure ou égale à 1.

12. Procédé suivant au moins l'une des revendications 5 à 11, **caractérisé en ce que** qu'on utilise de l'eau minéralisée pour la préparation de la solution aqueuse contenant des ions sulfate.

13. Procédé suivant au moins l'une des revendications 5 à 12, **caractérisé en ce que** la solution aqueuse contenant du dioxyde de chlore contient un phosphate soluble dans l'eau.
